(19) Europäisches Patentamt
European Patent Office
Office européen des brevets

(11) **EP 3 121 287 B1**

(12) **EUROPEAN PATENT SPECIFICATION**

(45) Date of publication and mention
of the grant of the patent:
**12.06.2024 Bulletin 2024/24**

(51) International Patent Classification (IPC):
**G06T 7/00** *(2017.01)* **G06T 7/62** *(2017.01)*
**H04N 13/20** *(2018.01)* **G01N 33/50** *(2006.01)*

(21) Application number: **14886334.3**

(22) Date of filing: **18.11.2014**

(52) Cooperative Patent Classification (CPC):
**G01N 33/5008; G06T 7/0012; G06T 7/62;**
**H04N 13/20;** G06T 2207/10101; G06T 2207/30024;
H04N 13/111

(86) International application number:
**PCT/JP2014/080438**

(87) International publication number:
**WO 2015/141059 (24.09.2015 Gazette 2015/38)**

(54) **DRUG EFFICACY EVALUATION METHOD AND IMAGE PROCESSING DEVICE FOR DRUG EFFICACY EVALUATION**

MEDIKAMENTENWIRKSAMKEITSAUSWERTUNGSVERFAHREN UND
BILDVERARBEITUNGSVORRICHTUNG ZUR MEDIKAMENTENWIRKSAMKEITSAUSWERTUNG

PROCÉDÉ D'ÉVALUATION DE L'EFFICACITÉ MÉDICAMENTEUSE ET DISPOSITIF DE
TRAITEMENT D'IMAGE SERVANT À LADITE ÉVALUATION DE L'EFFICACITÉ MÉDICAMENTEUSE

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **20.03.2014 JP 2014057594**

(43) Date of publication of application:
**25.01.2017 Bulletin 2017/04**

(73) Proprietor: **SCREEN Holdings Co., Ltd.
Kyoto-shi, Kyoto 602-8585 (JP)**

(72) Inventors:
• **FUJIMOTO, Hiroki
Kyoto-shi
Kyoto 602-8585 (JP)**
• **KOBAYASHI, Masayoshi
Kyoto-shi
Kyoto 602-8585 (JP)**
• **SASAKI, Ryuzo
Nagahama-shi
Shiga 526-0829 (JP)**

(74) Representative: **Kilian Kilian & Partner mbB
Zielstattstraße 23a
81379 München (DE)**

(56) References cited:
WO-A1-2009/107321     WO-A2-02/077903
JP-A- 2011 062 166     JP-A- 2012 202 761
JP-A- 2013 066 702     JP-A- 2013 068 477

• O.J. KLEIN ET AL: "Longitudinal, quantitative monitoring of therapeutic response in 3D in vitro tumor models with OCT for high-content therapeutic screening", METHODS, vol. 66, no. 2, 15 March 2014 (2014-03-15) , - 3 September 2013 (2013-09-03), pages 299-311, XP055424327, US ISSN: 1046-2023, DOI: 10.1016/j.ymeth.2013.08.028
• PONTUS LINDERHOLM ET AL: "Cell Culture Imaging Using Microimpedance Tomography", IEEE TRANSACTIONS ON BIOMEDICAL ENGINEERING, IEEE SERVICE CENTER, PISCATAWAY, NJ, USA, vol. 55, no. 1, 1 January 2008 (2008-01-01), pages 138-146, XP011198927, ISSN: 0018-9294, DOI: 10.1109/TBME.2007.910649

EP 3 121 287 B1

- Sriram Subramaniam ET AL: "Electron Tomography: a Powerful Tool for 3D Cellular Microscopy Technical refinements enable investigators to locate proteins inside cells and to anticipate tomographic reconstructions of bacteria", , 1 January 2008 (2008-01-01), XP055424356, Retrieved from the Internet: URL:https://www.asm.org/ccLibraryFiles/FIL ENAME/0000000316/nw20030061p.pdf [retrieved on 2017-11-13]
- Benedikt W. Graf ET AL: "Imaging and Analysis of Three-Dimensional Cell Culture Models" In: "Methods in Molecular Biology", 24 October 2009 (2009-10-24), Humana Press, Inc., US, XP055424361, ISSN: 1064-3745 vol. 591, pages 211-227, DOI: 10.1007/978-1-60761-404-3_13, * the whole document *
- SUSAN BRESLIN ET AL: "Three-dimensional cell culture: the missing link in drug discovery", DRUG DISCOVERY TODAY, vol. 18, no. 5-6, 1 March 2013 (2013-03-01), pages 240-249, XP55393379, AMSTERDAM, NL ISSN: 1359-6446, DOI: 10.1016/j.drudis.2012.10.003
- YONGJIN SUNG. ET AL.: 'Three-Dimensional Holographic Refractive-Index Measurementof Continuously Flowing Cells in a Microfluidic Channel' PHYSICAL REVIEW APPLIED February 2014, XP055224933

**Description**

TECHNICAL FIELD

The present invention relates to an efficacy evaluation method for evaluating a drug efficacy of a chemical substance upon a cell aggregate cultured in a culture medium.

BACKGROUND ART

In the technical field of drug discovery of developing a new pharmaceutical product, screening is carried out which aims at finding a drug which is efficacious on a particular cell which may for example be a cancer cell. One of such screening techniques is described in JP2011-062166A. During this screening, a chemical substance which is a drug candidate is administered to cultured cells and changes in the cells are observed. According to a conventional and general screening method, together with die candidate drug, a reagent which is indicative of a particular biochemical reaction due to an activity in the cells is added. As the quantity of a substance or light emission resulting from the biochemical reaction is measured, the level of the cell activity is determined. Known as such screening methods are for instance ATP assay, MTT assay, etc.

A problem with such screening methods is that among others, reagents are expensive, a relatively long period of time is necessary for a biochemical reaction to start and the reagents affect cell activities too much to conduct experiments in a chronological manner.

Meanwhile, as a method of observing without influencing a target cell, techniques for imaging the cell with a microscope or the like have been proposed. For example, according to the technique described in WO2009/107321, for the purpose of obtaining a stereoscopic image of a cell which is being cultured, the position of the focal point of a microscopic optical system is changed over multiple steps along the direction of depth and imaging is perfonned for every change. As a result, a pseudo three-dimensional image of the cell is obtained. This technique is suitable to imaging of a cell which is cultured (by plate culture) in a condition that the cell is adhered to the bottom surface of a container which contains a culture liquid.

Further prior art documents are

O.J. KLEIN ET AL, "Longitudinal, quantitative monitoring of therapeutic response in 3D in vitro tumor models with OCT for high-content therapeutic screening", METHODS, US, (20140315), vol. 66, no. 2,
PONTUS LINDERHOLM ET AL, "Cell Culture Imaging Using Microimpedance Tomography", IEEE TRANSACTIONS ON BIOMEDICAL ENGINEERING, IEEE SERVICE CENTER, PISCATAWAY, NJ, USA, (20080101), vol. 55, no. 1,
SRIRAM SUBRAMANIAM ET AL, "Electron Thmography: a Powerful Tool for 3D Cellular Microscopy Technical refinements enable investigators to locate proteins inside cells and to anticipate tomographic reconstructions of bacteria", (20080101),
BENEDICT W. GRAF ET AL, "Imaging and Analysis of Three-Dimensional Cell Culture Models", Methods in Molecular Biology, US, Humana Press, Inc., (20091024), vol. 591, pages 211 - 227, and
SUSAN BRESLIN ET AL, "Three-dimensional cell culture: the missing link in drug discovery", DRUG DISCOVERY TODAY, AMSTERDAM, NL, (20130301), vol. 18, no. 5-6.

CITATION LIST

PATENT LITERATURE

PTL1: JP2011-062166A
PTL2: WO2009/107321

SUMMARY OF INVENTION

TECHNICAL PROBLEM

In the recent years, screening which uses a three-dimensionally cultured cell aggregate is demanded for the purpose of improving the accuracy and the efficiency of efficacy evaluation. The reason is as follows. A lesion within a living body has a three-dimensional structure that a number of cells have been agglutinated. Therefore, the result of conventional efficacy evaluation using a plate-cultured cell does not often match with the efficacy inside a living body.

Because of this, screening which uses a cell aggregate is needed in order to evaluate in a closer condition to a living body.

For this purpose, it is desired that a technique would be established for realizing simple and convenient observation of changes in a three-dimensionally cultured cell aggregate caused by administration of a chemical substance. However, there have not been techniques which satisfy this requirement, including the conventional technique described above.

SOLUTION TO PROBLEM

The invention has been made in light of the problem above, and accordingly, the object of the invention is to provide a technique which makes it possible to more precisely evaluate how a chemical substance is efficacious upon a cell aggregate. This object is achieved by the subject-matter of claim 1. Further advantageous embodiments of the invention are the subject-matter of the dependent claims. Aspects of the invention are set out below.

One aspect of the invention is directed to an efficacy evaluation method for evaluating a drug as defined in claim 1.

According to thus configured invention, the drug efficacy of the chemical substance is evaluated in accordance with the feature amount of the cell aggregate which is obtained from the tomographic images along the vertical direction of the cell aggregate imaged along cross sections which are approximately vertical. The inventors of the invention identified the action of the chemical substance upon the cell aggregate as described below, and more details will be given later.

A cell aggregate of highly active cells has a shape of a relatively high degree of sphericity within a liquid such as a culture liquid. In the meantime, death of cells or deterioration of the activity level if any because of the efficacy of a chemical substance would give rise to a change such as shrinkage of the cell aggregate or deterioration of the degree of sphericity of the cell aggregate. According to the findings which the inventors of the invention obtained, such a change of the shape associated with collapse of the cell aggregate tends to occur in the bottom part of or below the cell aggregate. That is, a cell aggregate in which junctions among the cells cannot be maintained any more would start collapsing toward below because of the gravity, and dead cells would be separated from and fall off from the aggregate.

From the above, it is seen that what works effectively is imaging and observation of images of the cell aggregate from the side, and more preferably, along vertical-direction cross sections, instead of imaging of the top surface of the cell aggregate. Noting this, in this invention, the feature amount of the cell aggregate is calculated from tomographic images imaged along cross sections which approximately match with a vertical plane, and the efficacy of the chemical substance is evaluated based on the calculation result. In this fashion, it is possible to detect a change of the shape of the cell aggregate due to the action of the chemical substance without fail and accurately and efficiently evaluate the efficacy of the chemical substance upon the cell aggregate.

To achieve the object above, other aspect of the invention, not claimed, is directed to an image processing apparatus for evaluating a drug efficacy of a chemical substance upon a cell aggregate, comprising: an image acquiring device which acquires a plurality of tomographic images of the cell aggregate within a liquid imaged along cross sections which approximately match with a vertical plane; a stereoscopic image generator which generates a stereoscopic image of the surface of the cell aggregate based on the plurality of tomographic images; and feature amount calculator which calculates the feature amount of the cell aggregate based on the plurality of tomographic images or the stereoscopic image.

What has so far been possible through observation of an optical image of a cell aggregate for confirmation of the efficacy is at most acknowledgement of the presence or absence of growth or weakening through measurement of the size of the cell aggregate based on a two-dimensional image (an image of the cell aggregate imaged from above for instance). In contrast, the image processing apparatus according to the invention, not claimed, has a function of generating a stereoscopic image of a cell aggregate from a plurality of tomographic images along cross sections which are approximately along the vertical direction and calculating the feature amount of the cell aggregate. It is therefore extremely effective for execution of the drug efficacy evaluation method above. In other words, as the stereoscopic image of the cell aggregate is generated, it is possible to observe the cell aggregate from various field-of-view directions and quantitatively indicate the shape of the cell aggregate by the feature amount. Hence, it is possible to comprehensively evaluate the efficacy in accordance with the characteristics of the cell aggregate with respect to appearance or the feature amount. The image processing apparatus according to the invention, not claimed, can thus provide precise information to a user who wishes to evaluate the efficacy of the chemical substance, and can support the task in an extremely effective manner.

ADVANTAGEOUS EFFECTS OF INVENTION

With the efficacy evaluation method according to the invention, it is possible to detect a change of a cell aggregate due to the action of a chemical substance certainly and evaluate the efficacy of the chemical substance upon the cell aggregate precisely and efficiently. Further, with the image processing apparatus according to the invention, not claimed, it is possible to support those who perform such efficacy evaluation in an extremely effective fashion.

The above and further objects and novel features of the invention will more fully appear from the following detailed description when the same is read in connection with the accompanying drawing. It is to be expressly understood, however, that the drawing is for purpose of illustration only and is not intended as a definition of the limits of the invention.

BRIEF DESCRIPTION OF DRAWINGS

FIG 1 is a drawing which shows the image processing apparatus according to an embodiment of the invention.
FIGS. 2A is a drawing for describing the principle of imaging in the image processing apparatus.
FIGS. 2B is a drawing for describing the principle of imaging in the image processing apparatus.
FIG 3 is a flow chart which shows operations of this image processing apparatus.
FIGS. 4A is a drawing which shows an example of the tomographic images and the stereoscopic image.
FIGS. 4B is a drawing which shows an example of the tomographic images and the stereoscopic image.
FIG 5 is a flow chart which shows the drug efficacy evaluation method according to the embodiment.
FIGS. 6A is a drawing which shows an example of the weakening spheroid.
FIGS. 6B is a drawing which shows an example of the weakening spheroid.
FIGS. 6C is a drawing which shows an example of the weakening spheroid.
FIGS. 7A is a drawing which shows other example of the weakening spheroid.
FIGS. 7B is a drawing which shows other example of the weakening spheroid.
FIGS. 7C is a drawing which shows other example of the weakening spheroid.
FIGS. 7D is a drawing which shows other example of the weakening spheroid.
FIGS. 8A is a schematic drawing of vertical cross-sectional surface of the spheroid.
FIGS. 8B is a schematic drawing of vertical cross-sectional surface of the spheroid.
FIGS. 8C is a schematic drawing of vertical cross-sectional surface of the spheroid.

DESCRIPTION OF EMBODIMENTS

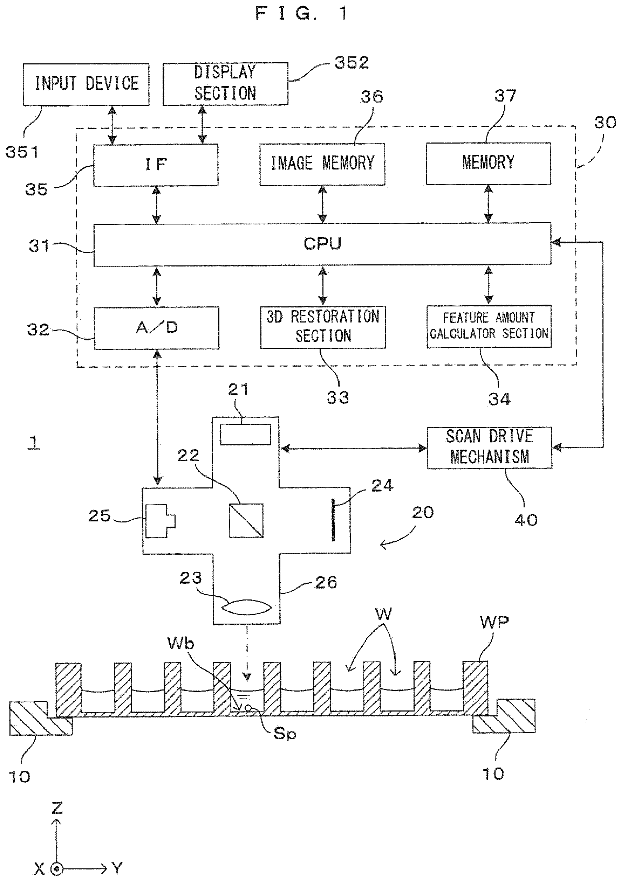

FIG 1 is a drawing which shows the image processing apparatus according to an embodiment of the invention. The image processing apparatus 1 can provide useful information for implementation of the drug efficacy evaluation method according to the invention. Because of this function, the image processing apparatus 1 can support execution of the drug efficacy evaluation method by a user in an extremely effective fashion. The structure of the image processing apparatus 1 and an embodiment of the drug efficacy evaluation method according to the invention which can be implemented using this apparatus will now be described in order. For unified presentation of the directions in drawings, the XYZ orthogonal coordinate axes are established as shown in FIG 1. The XY plane is a horizontal surface and the Z axis represents the vertical axis. In more detail, the (+Z) direction represents the vertically upward direction.

The image processing apparatus 1 images tomographic images of a spheroid (cell aggregate) cultured inside a liquid (which may for instance be a culture liquid). The image processing apparatus 1 processes thus obtained tomographic images and generates a stereoscopic image of the spheroid. Based on the tomographic images or the stereoscopic image, the image processing apparatus 1 calculates the feature amount which is quantitatively indicative of the characteristics of the spheroid with respect to appearance.

The image processing apparatus 1 comprises a holder section 10 which holds in an approximately horizontal posture a well plate (which is also called a "micro-plate") WP, in which a number of dents (wells) W which can hold a liquid at the top surface of a plate-like member, in such a manner that the openings of the wells W are directed toward above. Each well W of the well plate WP contains from the beginning a predetermined amount of an appropriate culture liquid, and a spheroid Sp is cultured in the liquid at the bottom surface Wb of the well W Although FIG 1 shows the spheroids Sp only in some wells W, the spheroid Sp is cultured in each one of the wells W

An imaging unit 20 is disposed above the well plate WP which is held by the holder section 10. The imaging unit 20 is capable of imaging tomographic images of a target object in a non-contact non-destructive (non-invasive) manner. As an example, use of an optical coherence tomography (OCT) apparatus will be described. The imaging unit 20 which is an OCT apparatus comprises a light source 21 which emits illumination light for a target object, a beam splitter 22 which splits light from the light source 21, an object lens 23, a reference mirror 24, a photo-detector 25 and a housing 26 which holds and houses them as one unit, as described in detail later.

Further, the image processing apparatus 1 comprises a control unit 30 which controls operations of the apparatus and a scan drive mechanism 40 which drives movable parts of the imaging unit 20. The control unit 30 comprises a CPU (Central Processing Unit) 31, an A/D convertor 32, a 3D restoration section 33, a feature amount calculator section 34, an interface (IF) section 35, an image memory 36 and a memory 37.

The CPU 31 governs operations of the entire apparatus by executing a predetermined control program, and the control program executed by the CPU 31 and data which are generated during processing are saved in the memory 37. The A/D convertor 32 converts a signal which the photo-detector 25 of the imaging unit 20 outputs in accordance with the amount of received light into digital image data. Based upon image data of a plurality of tomographic images photographed by the imaging unit 20, the 3D restoration section 33 generates a stereoscopic image (3D image) of the imaged cell aggregate. In accordance with the image data of one or a plurality of tomographic images imaged by the imaging unit 20 or the image data of the stereoscopic image generated by the 3D restoration section 33, the feature amount calculator section 34 calculates the feature amount which quantitatively expresses the morphological characteristics of the cell aggregate. The image memory 36 saves the image data of the tomographic images imaged by the imaging unit 20 and the image data of the stereoscopic image generated by the 3D restoration section 33.

The interface section 35 realizes communication between the image processing apparatus 1 and outside. More specifically, the interface section 35 has a function of communicating with external equipment, and a user interface function of accepting manipulation by a user and informing the user of various types of information. For this purpose, an input device 351 and a display section 352 are connected to the interface section 35. The input device 351 is for instance a key board, a mouse, a touch panel or the like which can accept manipulation and entry concerning selection of the functions of the apparatus, setting of operating conditions, etc. The display section 352 comprises a liquid crystal display for example which shows various types of processing results such as the tomographic images imaged by the imaging unit 20, the stereoscopic image generated by the 3D restoration section 33 and the feature amount calculated by the feature amount calculator section 34.

Further, the scan drive mechanism 40 makes the imaging unit 20 scan and move in accordance with a control command given from the CPU 31. As described next, the tomographic images of the cell aggregate which is the target object are obtained owing to combination of scan moving of the imaging unit 20 executed by the scan drive mechanism 40 and detection of the amount of the received light by the photo-detector 25.

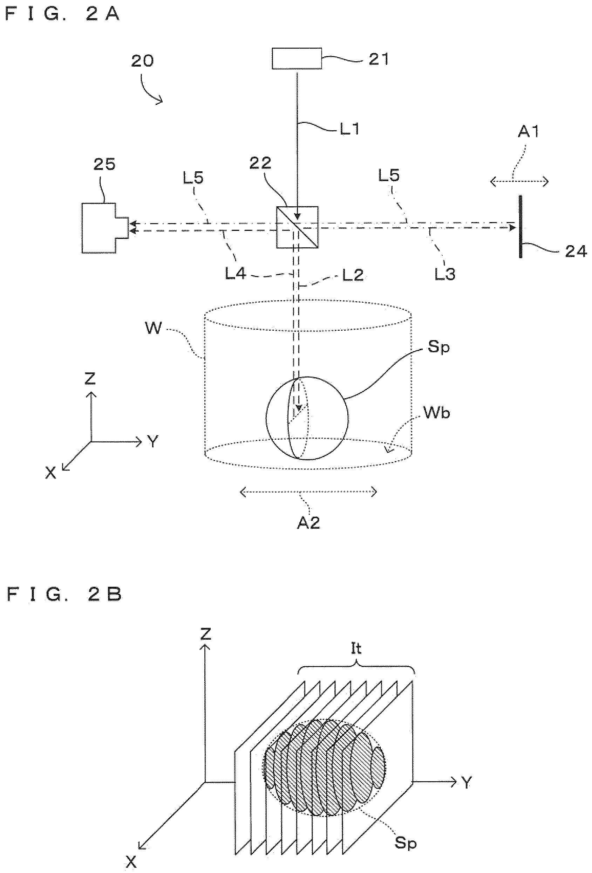

FIGS. 2A and 2B are drawings for describing the principle of imaging in this image processing apparatus. More specifically, FIG 2A is a drawing which shows optical paths inside the imaging unit 20, and FIG 2B is a schematic drawing which shows tomographic imaging of a spheroid. For easy understanding of the principle, FIG 2A omits the housing 26 and the object lens 23 which is equivalent to an ordinary object lens generally used in an imaging optical system among the respective structure elements of the imaging unit 20. As described earlier, the imaging unit 20 works as an optical coherence tomography (OCT) apparatus.

In the imaging unit 20, from the light source 21 which includes a light emitting element such as a laser diode or a light emitting diode for instance, a low-coherence light beam L1 is emitted. The light beam L1 impinges upon the beam splitter 22, and some light L2 indicated by the dotted-line arrow propagates toward the well W, and some light L3 indicated by the arrow of long dashed short dashed line propagates toward the reference mirror 24.

The light L2 propagating toward the well W impinges upon the spheroid Sp which is inside the culture liquid which is carried by the well W, and is reflected by the spheroid Sp. The light L2 is reflected at the surface of the spheroid Sp unless the spheroid Sp transmits the light beam L2. On the other hand, when the spheroid Sp has a property of transmitting the light beam L2 to a certain extent, the light beam L2 propagates into inside the spheroid Sp and is reflected by a structure element which is inside the spheroid. When the near infrared rays for instance are used as the light beam L2, it is possible to allow the incident light to reach even inside the spheroid Sp.

Light L4 reflected at the surface of or inside the spheroid Sp and light L5 reflected by the reference mirror 24 impinge upon the photo-detector 25 through the beam splitter 22. At this point, when the length of the optical path which is owing to reflection by the spheroid Sp and which is indicated by the dotted line is equal to the length of the optical path which is owing to reflection by the reference mirror 24 and which is indicated by the long dashed short dashed line, interference occurs between two light impinging upon the photo-detector 25. If the coherence length (coherent distance) of the light from the light source 21 is sufficiently short, of the reflection light from the spheroid Sp, only such reflection light from a reflection surface at a depth (Z-direction position) at which this reflection light has an optical path length corresponding to the optical path length of the reflection light from the reference mirror 24 interferes with the reflection light from the reference mirror 24.

As the photo-detector 25 detects the interference of the light, it is possible to selectively detect the reflection light from the reflection surface at the particular depth corresponding to the position of the reference mirror 24 from within the spheroid Sp. As the position of the reference mirror 24 is changed as indicated by the arrow A1, the reflection light from any desired depth inside the spheroid Sp can be detected. This is combined with scanning along the X direction of the light L2 impinging upon the well W, whereby the photo-detector 25 detects the interfering light at any time. This makes it possible to image tomographic images of the spheroid Sp along vertical-direction cross-sectional surfaces which are parallel to the XZ plane.

As indicated by the arrow A2, the relative position of the imaging unit 20 to the well W is changed along the Y direction over multiple steps, and a tomographic image is imaged for every change. As a result, as shown in FIG 2B, a number of tomographic images It of the spheroid Sp are obtained along cross-sectional surfaces which are parallel to the XZ plane. As the scan pitch in the Y direction is reduced, it is possible to obtain image data with sufficient resolution to grasp the stereoscopic structure of the spheroid Sp. Scan movements of the respective parts above in the imaging unit 20 are realized as the scan drive mechanism 40 operates after receiving a control command from the CPU 31.

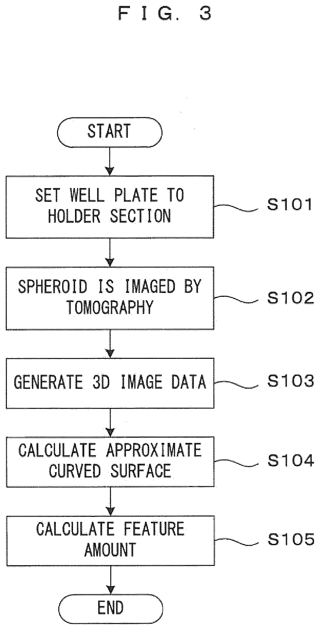

FIG 3 is a flow chart which shows operations of this image processing apparatus. At first, the well plate WP carrying the spheroid Sp to be imaged with the culture liquid is set to the holder section 10 by a user or a transportation robot (Step S101). The CPU 31 controls the imaging unit 20 and the scan drive mechanism 40 so that the spheroid Sp within the well W is imaged by tomography (Step S102).

Describing in more detail, scanning with a light beam changes the position at which the light beam impinges upon the spheroid Sp in the X direction. Further, as the position of the reference mirror 24 changes, the Z-direction position of a light receiving surface at which the reflection light is received is changed. Photo-detection is carried out together with this in a concerted manner, thereby acquiring tomographic images of the spheroid Sp along cross-sectional surfaces which are parallel surfaces to the XZ plane, that is, which is a vertical plane which is perpendicular to the Y direction. As the imaging unit 20 moves in Y direction relative to the well W, a tomographic image of the spheroid Sp is imaged along each cross-sectional surface while changing the Y-direction position of the cross-sectional surface. This is repeated, thereby obtaining a number of tomographic images at the cross-sectional surfaces which are at different positions from each other along the Y direction. The image data are saved in the image memory 36.

Based on thus obtained image data, the 3D restoration section 33 generates 3D image data corresponding to the stereoscopic image of the spheroid Sp (Step S103). Describing more specifically, as tomographic image data sporadically acquired along the Y direction are interpolated in the Y direction for instance, the 3D image data can be obtained. A technique of generating 3D image data from tomographic image data has already been practiced and will not be described in detail.

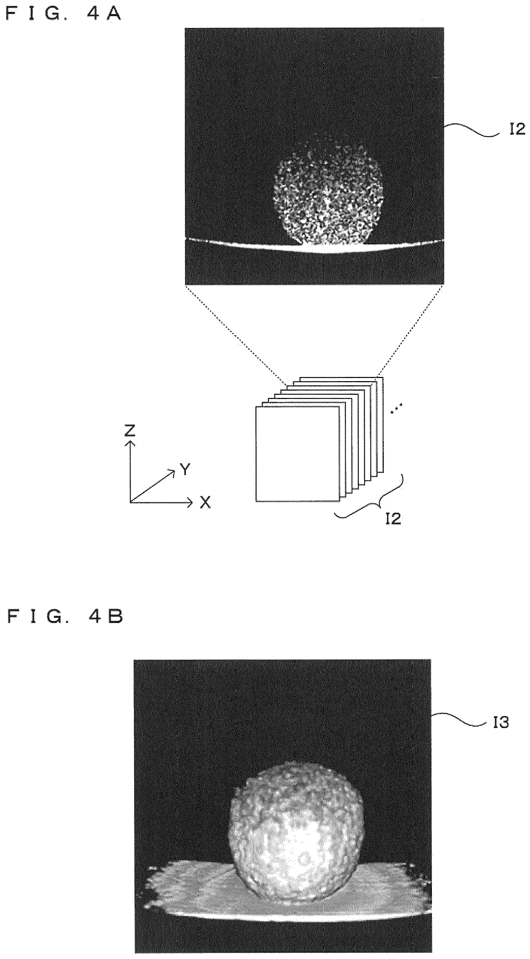

FIGS. 4A and 4B are drawings which show an example of the tomographic images and the stereoscopic image. From the number of tomographic images (two-dimensional images) 12 (FIG 4A) of the spheroid Sp imaged along cross sections parallel to the XZ plane while changing the position in the Y direction, the stereoscopic image (three-dimensional image) I3 (FIG 4B) representing the total appearance of the spheroid Sp is created. The tomographic images 12 are the examples in FIG 4A clearly show the surface of the spheroid Sp, namely, the interface between the interior of the spheroid Sp and the culture liquid. It also shows structures inside the spheroid Sp, i.e., fine textures corresponding to the interfaces among many cells which form the spheroid Sp. Meanwhile, the stereoscopic image in FIG 4B clearly shows the shape of the surface of the spheroid Sp.

The arc-like white stripe at the bottom part of the image in FIG 4A is the image of the bottom surface Wb of the well W The bottom surface Wb of the well W, having a slightly concave shape toward the center, shows itself as such an arc-like image. This is similar with the white plate-like image in the bottom part of the image in FIG 4B. This is similar in the later drawings as well.

The 3D image data thus generated horn the tomographic images represent correlation between the coordinates of the respective pixels in a virtual XYZ pixel space and their pixel values. Such 3D image data, once generated, can be used to perform various types of processing. For example, through image processing, images which correspond to the images of the spheroid Sp seen from various field-of-view directions may be created and displayed by the display section 352. When this is done, a user can observe the external shape, the surface shape and the like of the spheroid as if the spheroid were right in front of the user and viewed from a desired direction.

The operations of the image processing apparatus 1 will now be continuously described with reference back to FIG 3. As indicated by the example in FIG 4B, there are fine irregularities which correspond to the interfaces among the cells on the surface of the spheroid Sp. As described later, the drug efficacy evaluation method according to the invention requires to determine based upon characteristics of the spheroid Sp with respect to overall shape. The fine irregularities could therefore cause an error in quantitative representation of the characteristics of the spheroid Sp. Noting this, an approximate curved surface is calculated which is approximation of the surface of the spheroid Sp by a simpler, i.e., less irregular curved surface (Step S104). Various types of approximation calculation methods are applicable for this, one of which will be described later.

The calculated approximate curved surface is a curved surface which expresses the envelop contour of the spheroid Sp. Although this approximate curved surface does not contain much information concerning the conditions of the individual cells which form the spheroid Sp, this approximate curved surface is more clearly indicative of the characteristics of the spheroid Sp with respect to overall shape. Based upon this approximate curved surface, the feature amount calculator section 34 calculates the feature amount which quantitatively expresses the characteristics of the spheroid Sp (Step S105).

Where the drug efficacy evaluation method according to the invention described later is implemented, the efficacy of a chemical substance which is a drug candidate is evaluated in accordance with how the shape of a spheroid Sp administered with the chemical substance changes. To note in particular, a normal spheroid would have a nearly spherical shape within a culture liquid, whereas a spheroid damaged by the chemical substance would have a shrank or deteriorated shape. A feature amount is therefore used with which it is possible to quantitatively detect such a change of the external shape. Calculated as feature amounts are for instance the diameter, the volume and the surface area size of the spheroid, the curvature and the radius of curvature of the surface of the spheroid, and the degree of sphericity of the spheroid. As the feature amounts are calculated using an approximate curved surface, calculation errors attributable to the condition of the surface of the spheroid can be reduced.

One example of the approximate curved surface calculation methods mentioned above will now be described. The approximate curved surface in the XYZ pixel space may be expressed by the equation z = f (x, y), and the function f may be calculated based upon 3D image data. For the purpose of calculating a smooth curved surface from 3D image data, least square approximation is performed. As a simple and easy example, use of plane approximation, namely, the formula below will now be described:

$$z = ax + by + c \qquad \dots (1)$$

Such values of the constants a, b and c are calculated which minimize the sum of squares of a difference between the z value and z; which are calculated by substituting $x_i$ and $y_i$ in the numerical expression (1) above in the presence of the sequence of points $(x_i, y_i, z_i)$ where i = 1 to n (n is a natural number). Describing more specifically, the formula of least sum of squares is solved simultaneously with an equation which uses 0 as the value resulting from a formula which is solved by partial differentiation using the constants a, b and c as variables.

The following is an example of how the formula can be solved using a determinant. When a vector Z expresses a set of the z values which are obtained by substituting $x_i$ and $y_i$ in the numerical expression (1), the formula below expresses the vector Z.

[Formula 1]

$$Z = \begin{bmatrix} 1 & x1 & y1 \\ \vdots & \vdots & \vdots \\ 1 & xn & yn \end{bmatrix} \begin{pmatrix} c \\ a \\ b \end{pmatrix} \qquad \dots (2)$$

The formula below is defined and an unknown vector X is calculated.
[Formula 2]

$$G = \begin{bmatrix} 1 & x1 & y1 \\ \vdots & \vdots & \vdots \\ 1 & xn & yn \end{bmatrix}, \quad X = \begin{pmatrix} c \\ a \\ b \end{pmatrix} \qquad \dots (3)$$

Since the coefficient matrix G is a rectangular matrix, computation is complicated. Therefore, the transposed matrix $^tG$ is applied to the both sides of the numerical expression (2) from the right-hand side, and the following is obtained:

$$^tGZ = {}^tGG \cdot X \qquad \dots (4)$$

The unknown vector X can then be expressed by the following:

$$X = ({}^tGG)^{-1} \cdot {}^tGZ \qquad \dots (5)$$

The matrix $^tGG$ is a normal matrix, i.e., a square matrix. The right-hand side of the numerical expression (5) can be solved using Gaussian elimination for example.

[Formula 3]

$$^tGZ = \begin{bmatrix} 1 & \cdots & 1 \\ x1 & \cdots & xn \\ y1 & \cdots & yn \end{bmatrix} \begin{pmatrix} z1 \\ \vdots \\ zn \end{pmatrix} = \begin{pmatrix} \sum zi \\ \sum xizi \\ \sum yizi \end{pmatrix} \qquad \dots (6)$$

$$^tGG = \begin{bmatrix} 1 & \cdots & 1 \\ x1 & \cdots & xn \\ y1 & \cdots & yn \end{bmatrix} \begin{bmatrix} 1 & x1 & y1 \\ \vdots & \vdots & \vdots \\ 1 & xn & yn \end{bmatrix} = \begin{bmatrix} n & \sum xi & \sum yi \\ \sum xi & \sum xi^2 & \sum xiyi \\ \sum yi & \sum xiyi & \sum yi^2 \end{bmatrix} \qquad \dots (7)$$

From these results, the unknown vector X is calculated and the values which the constants a, b and c take are calculated. Substituting them in the numerical expression (1), the equation expressing the approximate curved surface is obtained.

While the foregoing has described plane approximation by a linear equation, similar thinking applies even to an equation of a higher degree.

In the case of a quadratic equation for instance, the following is used:

$$z = f(x, y) = ax^2 + by^2 + cxy + dx + ey + f \qquad \dots (8)$$

An equation is established, using six constants a through f which are unknown and using a coefficient matrix of n rows and six columns in which values 1, $x_i$, $y_i$, $x_iy_i$, $x_i^2$ and $y_i^2$ are elements of one row instead of using the coefficient matrix G which is in the numerical expression (2).

Using a transposed matrix in a similar fashion to the above, this is converted into an equation which has a 6x6 normal matrix, thereby calculating the unknown values a through f.

Next, how to calculate the curvature of the approximate curved surface at a point P which is on thus calculated approximate curved surface will be described. The curvature of the curved surface should be expressed using both the Gaussian curvature K and the plane curvature H. The parameters below are set for the curved surface equation z = f (x, y).

[Formula 4]

$$p = \frac{\partial f}{\partial x}, \quad q = \frac{\partial f}{\partial y}, \quad r = \frac{\partial^2 f}{\partial x^2}, \quad s = \frac{\partial^2 f}{\partial x \partial y}, \quad t = \frac{\partial^2 f}{\partial y^2} \qquad \dots (9)$$

This allows the numerical expression below to define the Gaussian curvature K and the plane curvature H. When the curvature is to be calculated within a pixel space in which coordinates are sporadically expressed in the unit of pixels, differentiation in the numerical expression (9) may be replaced with a difference of pixel pitches and numerical calculation may be performed.

[Formula 5]

$$K = \frac{rt - s^2}{\left(1 + p^2 + q^2\right)^2}, \quad H = \frac{r\left(1 + q^2\right) - 2pqs + t\left(1 + p^3\right)}{2\left(1 + p^2 + q^2\right)^{3/2}} \qquad \dots (10)$$

The method of evaluating the drug efficacy of a chemical substance using the image processing apparatus 1 having the above structure will now be described. A conventional way is to administer a chemical substance which is a drug candidate to a target cell two-dimensionally cultured in a culture liquid, observe how the viability of the cell changes and evaluate the efficacy of the chemical substance. However, the recent years have seen instances that a chemical substance whose efficacy was thus confirmed do not exhibit similar in-vivo efficacy. It is considered one of the causes is that while a number of target cells cluster into an aggregate inside a living body and have a three-dimensional structure, the efficacy is confirmed inside a two-dimensionally cultured cell. That is, in the case of two-dimensionally cultured cells,

EP 3 121 287 B1

an administered chemical substance comes into contact with many cells and the efficacy would easily manifest itself, whereas in the case of cells which have a three-dimensional structure, the chemical substance would not easily reach those cells which are inside the aggregate and would not easily show the efficacy.

It is therefore more necessary than before to evaluate the efficacy of a chemical substance which is a drug candidate using an aggregate of target cells which were three-dimensionally cultured in a culture liquid. However, a technique has not been established which makes it possible to closely observe the condition of the cell aggregate which has such a three-dimensional structure, i.e., a spheroid which is inside a culture liquid. An environment for precise and efficient evaluation has not been built yet. The image processing apparatus 1 above is suitable to such observation, and utilizing this, it is possible to more precisely and efficiently perform efficacy evaluation (screening) of the chemical substance.

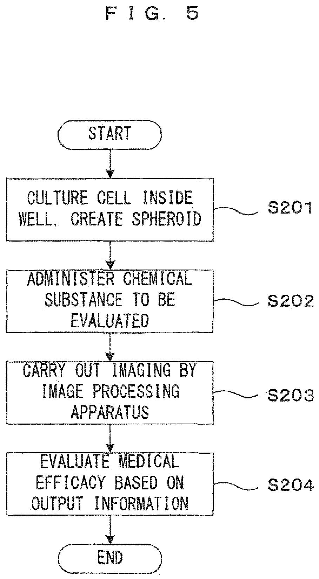

FIG 5 is a flow chart which shows the drug efficacy evaluation method according to this embodiment. First, the culture liquid is poured as needed into each well W of the well plate WP, cells which are targets are cultured inside the culture liquid and the spheroids Sp are created (Step S201). The chemical substance which needs be evaluated is administered in a predetermined amount into each well W (Step S202).

The image processing apparatus 1 turns the spheroids Sp thus administered with the chemical substance into image data (Step S203). In short, the image processing apparatus 1 performs tomographic imaging and computation based upon the resulting image data. Imaging may be carried out only once after a predetermined period of time from administration of the chemical substance. Alternatively, what is known as time lapse imaging may be executed which is imaging over multiple times at constant time intervals. The image processing apparatus 1 calculates the tomographic image data, the stereoscopic image data and the feature amounts of the spheroids Sp, following which the efficacy of the chemical substance is comprehensively evaluated based on this information (Step S204).

In the presence of the efficacy, a spheroid Sp would be weakened and shrink. Hence, if the feature amounts calculated from the respective images captured at time intervals are indicative of a decrease with time of the diameter, the surface area size or the volume of the spheroid Sp, it is determined that the efficacy is confirmed. If the feature amounts do not represent a significant change or indicates an increase, it is determined that the efficacy is missing. The volume of the spheroid Sp can be calculated by integrating the cross sectional area size of the spheroid Sp which was taken along a certain cross-sectional direction in the perpendicular direction to the cross-sectional direction. Calculation directly from a plurality of tomographic images acquired through imaging is also possible for example, instead of using three-dimensional images. After calculating the volume V, the radius r of a sphere which has the same volume as that of the spheroid Sp can be calculated from the relationship below:

$$V = 4\pi r^3/3$$

The value r can be regarded as the radius of curvature, in which case the curvature is expressed as $(1/r)$.

However, as described below, it is difficult to determine the viability of the spheroid Sp only from these pieces of information in some instances.

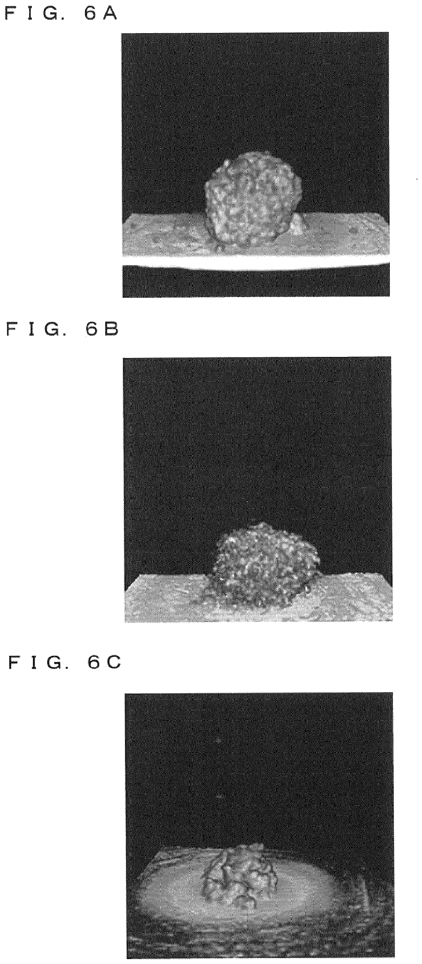

FIGS. 6A through 6C are drawings which show an example of weakening spheroids. In this illustrated example in the drawings, the field-of-view direction is set as a direction of looking down on the spheroid from slightly above the side. FIG 6A shows the image (stereoscopic image) of a spheroid which exhibits relatively high viability and has an approximately spherical shape formed by a number of cells. However, a sign of collapse is seen in the right bottom portion of the image.

Meanwhile, FIG 6B shows the image of a spheroid which was weakened and started to break down and FIG 6C shows the image of a spheroid which further collapsed. In these examples, junctions among cells became too weak to maintain the spherical shapes, and the cells form irregularly-shaped aggregates. In the case of these, it may sometimes be difficult to distinguish from the condition shown in FIG 6A based only on the feature amounts such as the surface area sizes and the volumes or on observation from above. From a qualitative perspective, it nevertheless is relatively easy to discover deterioration of the shapes by observing the stereoscopic images from various field-of-view directions. From a quantitative perspective, it is possible to detect collapse from the spherical shapes by observing changes of the feature amounts such as the curvature, the degree of sphericity and the like of the surfaces of the spheroids.

The surface (or its approximate curved surface) of the spheroid Sp is not a perfect spherical surface. For the purpose of sensing collapse of the shape therefore, it is effective to compare the curvatures taken along two or more mutually different cross-sectional surfaces with each other. The spheroid Sp in which the viability of the cells has dropped down would collapse toward below, i.e., as if it were caving in toward the bottom surface of the well W, due to the gravity. It is therefore considered that the curvature of the surface as it is when the spheroid Sp is viewed from the horizontal direction changes to a particularly large extent. From this, it is possible to determine that the spheroid Sp is collapsing, that is, the efficacy is shown when there is a significant difference between the curvature Rxy viewed from the horizontal direction (i.e., on the XY plane) and the curvature Rxz viewed from the vertical direction (i.e., on the XZ plane for instance).

For confirmation of this from displayed images, it is effective to observe the spheroid Sp particularly from a close direction to the horizontal direction.

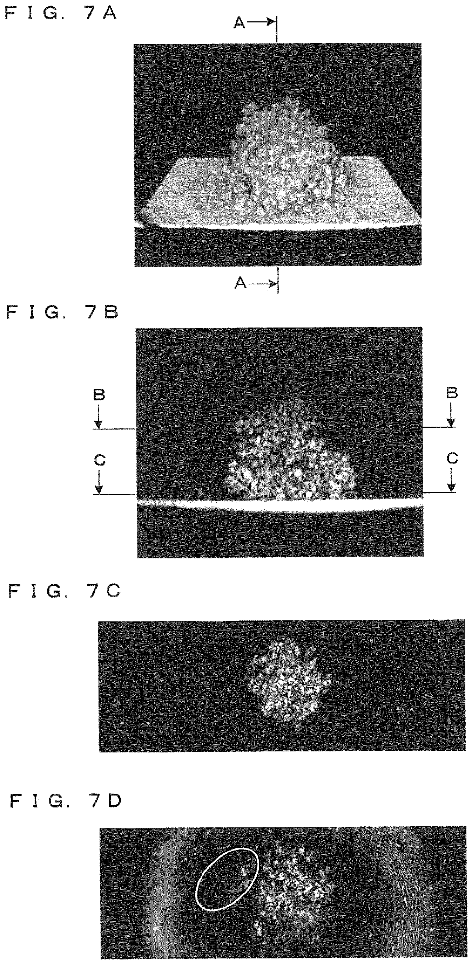

FIGS. 7A through 7D are drawings which show other examples of weakening spheroids. FIG 7A shows the example of a different stereoscopic image of the spheroid which has started to collapse. FIG 7B is a cross-sectional view of FIG 7A taken along the arrow line A-A and corresponds to an image of the spheroid viewed from the horizontal direction along a cross-sectional surface which is a vertical plane. While the spheroid still maintains a shape which is relatively close to a spherical shape in this example, particle-like matters are scattered as if to surround the spheroid on the bottom surface Wb of the well.

FIG 7C and FIG 7D are cross-sectional views of FIG 7B taken along the arrow line B-B and the arrow line C-C, respectively, showing the spheroids along horizontal cross-sectional planes. In FIG 7C along a horizontal cross-sectional plane which is relatively far from the bottom surface Wb of the well, the periphery of the spheroid has a shape which is relatively close to a round shape. In contrast, in FIG 7D along a horizontal cross-sectional plane which is close to the bottom surface Wb of the well, there is an unclear image of an unstable shape at a position enclosed by the white circle for example around the spheroid which is a cluster at the center. This is also an image of the particle-like matters which are dispersed at the bottom surface of the well. In FIG 7D, the arc-like white area spreading as if to surround the spheroid at a far position from the spheroid is the reflection of a part of the curved bottom surface Wb of the well.

The particle-like matters distributed over the bottom surface Wb of the well in these images are free cells, or residues (debris) of the cells, which have fallen off from the spheroid and settled down and deposited on the bottom of the well W Cells near the surface of the spheroid Sp may become incapable of staying at their positions and fall off, which is a phenomenon attributable to reduction of the viability of the cells by a chemical substance. Thus freed cells have a low level of activity, precipitate at the bottom of the culture liquid and built up on the bottom surface Wb of the well. Therefore, the presence or absence and the amounts of free cells and debris can be utilized as effective information which is indicative of the efficacy. The presence or absence of free cells and the like can be determined by visual observation of images which the display section 352 displays.

Alternatively, detection of free cells and the like may be automated through image processing. For instance, from the size, the shape and the like of the range of cell distribution in the image as that shown in FIG 7D along the horizontal cross-sectional surface which is close to the bottom surface Wb of the well, it is possible to detect the presence or absence, the amount and the like of free cells. This is because while cells would be agglutinated over a relatively small range inside a spheroid, free cells would stay dispersed without clustering together.

A change in a spheroid is examined comprehensively from these pieces of information, namely, the results of visual observation of the spheroid from various field-of-view directions based on a stereoscopic image reconstructed from tomographic images, various types of calculated feature amounts, the result of detection of free cells, etc. As compared with conventional screening techniques which are dependent upon subjective decisions or would damage cells therefore, it is possible to evaluate the efficacy more precisely and efficiently. This allows more efficient screening of various types of chemical substances. Use of tomographic images imaged along cross-sectional surfaces which approximately match with a vertical plane for evaluation makes this effect remarkable. The reason will now be described.

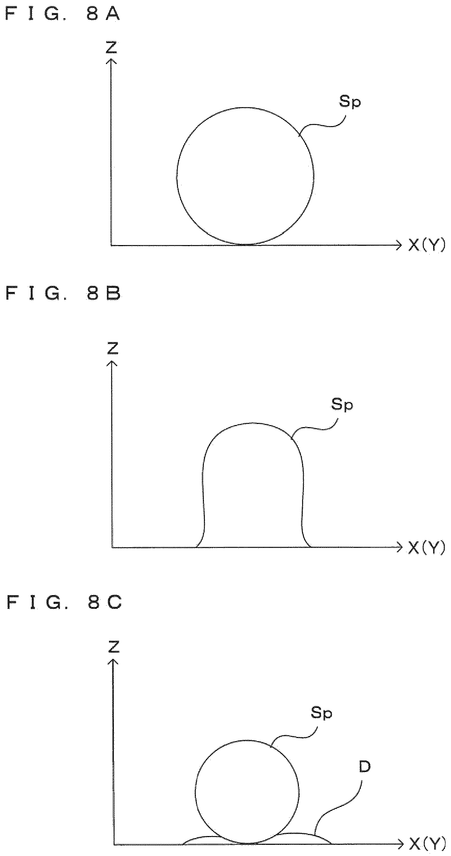

FIGS. 8A through 8C are schematic drawings of vertical cross-sectional surfaces of spheroids. FIG 8A shows a spheroid Sp which exhibits high viability and has an approximately round shape in its vertical cross section. In a similar manner, its external shape is approximately round even when this spheroid Sp is imaged downwardly from above or even when this spheroid Sp is imaged in horizontal cross section by tomography. FIG 8B shows a spheroid Sp which has partially collapsed, and the collapse-induced changes of the shape are apparent in the bottom part of the spheroid Sp. Through observation from above (the Z-axis direction) or in horizontal cross section (the XY plane), such changes are behind the spheroid and difficult to be discovered. However, use of tomographic images along vertical cross section makes it possible to easily find such changes of the shape of the spheroid Sp.

FIG 8C shows an instance that free cells, debris and the like have settled down on the bottom surface Wb of the well. As described earlier, many free cells D separated from a spheroid Sp is one representation of the efficacy of an administered chemical substance. When merely observing the shape of the spheroid Sp from outside, one may overlook such free cells D. Based upon two-dimensional images photographed from above in particular, it is difficult to distinguish cells which form the spheroid Sp from the free cells D.

However, during the process of acquiring tomographic images along vertical planes, it is possible to capture into images the free cells D, debris and the like which settle down around but yet with a distance from the spheroid Sp (particularly a bottom part). It is therefore possible to avoid such overlooking. Particularly when observing a stereoscopic image reconstructed from tomographic images from various directions, one can easily discover not only the spheroid Sp but structure elements which are distributed around the spheroid Sp as well.

As illustrated above, in the image processing apparatus 1 of the embodiment, the imaging unit 20 functions as the "image acquiring device" of the invention. Further, the 3D restoration section 33 and the feature amount calculator section 34 function as the "stereoscopic image generator" and the "feature amount calculator" of the invention, respectively. Further, the well plate WP corresponds to the "container" of the invention and the holder section 10 functions as the "holder" of the invention. Meanwhile, the display section 352 functions as the "display device" of the invention.

The invention is not limited to the embodiment described above but may be modified in various manners in addition to the embodiment above, to the extent not deviating from the invention as defined in the appended claims. For instance, the feature amounts which are calculated according to the embodiment above are merely some examples of what indicate characteristics of a spheroid with respect to shape. The invention is not limited to use of these feature amounts. That is, only some of the above feature amounts may be used, or other feature amounts than those described above may be used.

Further, in the control unit 30 according to the embodiment above, the CPU 31, the 3D restoration section 33 and the feature amount calculator section 34 are individual function blocks for instance. Instead, the 3D restoration section 33 and the feature amount calculator section 34 may be one unified GPU (Graphic Processing Unit). Alternatively, these functions may be realized by a single CPU.

Further, for example, an optical coherence tomography (OCT) apparatus is used as the imaging unit 20 which performs tomographic imaging according to the embodiment above. However, the "image acquiring means" of the embodiment may be an imaging apparatus which uses other imaging principles and is capable of tomographic imaging of a spheroid in a non-destructive manner, e.g., a con-focal microscopic imaging apparatus. An optical coherence tomography apparatus as that used in the embodiment above is more advantageous as it can complete imaging in a shorter period of time.

Further, for example, while the embodiment above uses an imaging apparatus as the "image acquiring means" of the embodiment, the image processing apparatus according to the invention, not claimed, is not necessarily required to have an imaging function. In other words, it may only receive tomographic image data captured by an external imaging apparatus and perform image processing. In this case, the interface section for receiving image data from outside serves as the "image acquiring means" of the invention.

The invention is applicable to a screening technique for discovering a chemical substance which is efficacious upon a particular cell. It is possible to precisely evaluate the efficacy upon a cell aggregate which is three-dimensional aggregation of cells, which can be utilized in drug discovery of finding a drug which has an effective in-vivo action.

In addition, as described above, the invention may further comprise detecting a free cell which has fallen off from a cell aggregate and settled down on a bottom surface of the container based on tomographic images and determine the efficacy of a chemical substance based on the calculation results of the feature amount and the detection result of the free cell. Since dead cell falls off from a cell aggregate and settle down at the bottom of the container, the presence of such free cell can be powerful evidence of the efficacy of the chemical substance. Hence, instead of noting only the shape of the cell aggregate, detection and evaluation of the presence or absence, the amount and the like of free cells which have settled down around the cell aggregate and particularly at the bottom surface of the container makes it possible to further improve the accuracy.

Further, tomographic images may be acquired along mutually different cross sections for instance in the invention. Since the shape of a cell aggregate is not a perfect sphere, evaluation using the plurality of tomographic images further improves the evaluation accuracy.

In this case, for instance, a stereoscopic image of the surface of the cell aggregate may be generated by image processing based on the plurality of tomographic images. As many tomographic images are collected, a pseudo-stereoscopic image of the cell aggregate can be obtained. When the stereoscopic image of the cell aggregate is generated from the tomographic images, it is possible to observe the shape of the cell aggregate, the condition of the surface and the like for example from various field-of-view directions. In combination with the calculation result of the feature amount, this realizes comprehensive evaluation, which aims at improvement of the evaluation accuracy.

What can be used as further feature amounts used in the invention is, for example, at least one of the surface area size of the cell aggregate, the volume of the cell aggregate, the curvature of the surface of the cell aggregate and the radius of curvature of the surface of the cell aggregate. From the surface area size and the volume of the cell aggregate, it is possible to know the size of the cell aggregate. Further, from the curvature and the radius of curvature of the surface of the cell aggregate, it is possible to know the shape of the surface of the cell aggregate. Any one of these can be used as information for determining whether the cell aggregate is growing or getting weakened.

In the event that the curvatures of the surface of the cell aggregate taken along mutually different cross sections are included as the feature amounts, as they are compared with each other, it is possible to determine whether the cell aggregate still maintains a spherical shape or the shape has deteriorated.

Further, for instance, the invention may require to perform tomographic imaging of the cell aggregate a plurality of times at predetermined time intervals and determine the efficacy of the chemical substance based on changes with time of the feature amount calculated from tomographic images acquired through imaging. As the changes with time of the cell aggregate are studied, it is possible to more precisely determine the efficacy of the chemical substance. There already are practical tomographic techniques which achieve non-contact non-destructive imaging of a target object, e.g., optical coherence tomographic techniques. When they are implemented, it is possible to perform imaging without affecting the cell aggregate. This makes it possible to observe changes with time of the cell aggregate.

Further, in the invention, for instance, the feature amount calculator may calculate the feature amount concerning an approximate curved surface corresponding to the surface of the cell aggregate obtained based on the stereoscopic image. The cell aggregate is aggregation of many cells, and on its surface, there are uneven irregularities which correspond to the surfaces of the individual cells. Those fine irregularities do not represent characteristics of the entire cell aggregate. Therefore, the feature amount may be calculated on approximation by simpler curved surface, which attains more precise quantification of the characteristics of the cell aggregate with respect to shape.

Further, for example, the invention may further comprise a holder which holds a container which carries a liquid in which the cell aggregate is contained, and the image acquiring device may comprise an imager which performs tomographic imaging of the cell aggregate within the container. While tomographic images may be imaged by an external imaging apparatus, in the event the image processing apparatus of the invention, not claimed, comprises the holder which holds the container and the imager, it is possible to acquire tomographic images which best suit the purpose of efficacy evaluation. As an imager which achieves such imaging, an optical coherence tomography apparatus for instance may be used as described earlier.

Further, for example, the invention may comprise displaying means which is equipped with a function of displaying a stereoscopic image and is capable of changing the field-of-view direction toward the cell aggregate in the displayed image. Such configuration allows to provide to a user various information concerning the characteristics of the cell aggregate with respect to appearance, making it possible for the user to comprehensively evaluate the efficacy from the displayed image and the calculated feature amount. Although the invention has been described with reference to specific embodiments, this description is not meant to be construed in a limiting sense. The invention is defined by the appended claims.

REFERENCE SIGNS LIST

| 1 | image processing apparatus |
| 10 | holder section (holder) |
| 20 | imaging unit (image acquiring device, optical coherence tomography imager) |
| 21 | light source |
| 22 | beam splitter |
| 24 | reference mirror |
| 25 | photo-detector |
| 30 | control unit |
| 33 | 3D restoration section (stereoscopic image generator) |
| 34 | feature amount calculator section (feature amount calculator) |
| 352 | display section (display device) |
| Sp | spheroid (cell aggregate) |
| W | well |
| WP | well plate |

**Claims**

1.  A computer-implemented efficacy evaluation method for evaluating a drug efficacy of a chemical substance upon a cell aggregate, comprising:

    acquiring a plurality of tomographic images of the cell aggregate which are imaged along cross sections matching with a vertical plane where the cell aggregate is held inside a liquid which is contained in a container and has added the chemical substance;
    generating a three-dimensional image of a surface of the cell aggregate by image processing based on the plurality of tomographic images; and
    calculating a feature amount of the cell aggregate as information for determining the drug efficacy of the chemical substance based on the three-dimensional image, the feature amount including a curvature of a surface of the cell aggregate in a cross section viewed from a horizontal direction and a curvature of a surface of the cell aggregate in a cross section viewed from a vertical direction,
    wherein:

        tomographic imaging of the cell aggregate is performed a plurality of times at predetermined time intervals;

the drug efficacy of the chemical substance is determined based on changes with time of the feature amounts calculated from tomographic images acquired through the imaging; wherein
it is determined that the drug efficacy is shown on the basis of a difference between the curvature of a surface of the cell aggregate in a cross section viewed from the horizontal direction and the curvature of a surface of the cell aggregate in a cross section viewed from the vertical direction.

2. The efficacy evaluation method of claim 1, further comprising

detecting a free cell which has fallen off from the cell aggregate and settled down on a bottom surface of the container based on tomographic images,
wherein the efficacy of the chemical substance is determined based on a calculation result of the feature amount and a detection result of the free cell.

**Patentansprüche**

1. Computerimplementiertes Wirksamkeitsbewertungsverfahren zur Bewertung der Arzneimittelwirksamkeit einer chemischen Substanz auf ein Zellaggregat, aufweisend:

Erfassen einer Vielzahl von tomographischen Bildern des Zellaggregats, die entlang von Querschnitten aufgenommen werden, die mit einer vertikalen Ebene übereinstimmen, in der das Zellaggregat in einer Flüssigkeit gehalten wird, die in einem Behälter enthalten ist und der die chemische Substanz hinzugefügt wurde;
Erzeugen eines dreidimensionalen Bildes einer Oberfläche des Zellaggregats durch Bildverarbeitung auf der Grundlage der mehreren tomographischen Bilder; und
Berechnen eines Merkmalsbetrags des Zellaggregats als Information zur Bestimmung der Arzneimittelwirksamkeit der chemischen Substanz auf der Grundlage des dreidimensionalen Bildes, wobei der Merkmalsbetrag eine Krümmung einer Oberfläche des Zellaggregats in einem Querschnitt, betrachtet aus einer horizontalen Richtung, und eine Krümmung einer Oberfläche des Zellaggregats in einem Querschnitt, betrachtet aus einer vertikalen Richtung, umfasst,
wobei:

die tomographische Abbildung des Zellaggregats wird mehrmals in vorbestimmten Zeitabständen durchgeführt;
die Arzneimittelwirksamkeit der chemischen Substanz auf der Grundlage von zeitlichen Änderungen der Merkmalsmengen bestimmt wird, die aus den durch die Bildgebung gewonnenen tomographischen Bildern berechnet werden; wobei
wird festgestellt, dass die Wirksamkeit des Arzneimittels auf der Grundlage des Unterschieds zwischen der Krümmung einer Oberfläche des Zellaggregats in einem Querschnitt in horizontaler Richtung und der Krümmung einer Oberfläche des Zellaggregats in einem Querschnitt in vertikaler Richtung angezeigt wird.

2. Wirksamkeitsbewertungsverfahren nach Anspruch 1, ferner aufweisend

Erkennen einer freien Zelle, die aus dem Zellaggregat herausgefallen ist und sich auf einer Bodenfläche des Behälters niedergelassen hat, auf der Grundlage von tomographischen Bildern,
wobei die Wirksamkeit der chemischen Substanz auf der Grundlage eines Berechnungsergebnisses der Merkmalsmenge und eines Nachweisergebnisses der freien Zelle bestimmt wird.

**Revendications**

1. Procédé d'évaluation de l'efficacité mis en œuvre par ordinateur pour évaluer l'efficacité médicamenteuse d'une substance chimique sur un agrégat cellulaire, comprenant :

d'acquérir une pluralité d'images tomographiques de l'agrégat cellulaire qui sont prises le long de sections transversales correspondant à un plan vertical où l'agrégat cellulaire est maintenu à l'intérieur d'un liquide contenu dans un récipient, et ajouté à la substance chimique ;
de générer une image tridimensionnelle d'une surface de l'agrégat cellulaire par traitement d'image sur la base de la pluralité d'images tomographiques ; et

de calculer une quantité de caractéristiques de l'agrégat cellulaire sous la forme d'informations pour déterminer l'efficacité médicamenteuse de la substance chimique sur la base de l'image tridimensionnelle, la quantité de caractéristiques comportant une courbure d'une surface de l'agrégat cellulaire dans une section transversale vue depuis une direction horizontale et une courbure d'une surface de l'agrégat cellulaire dans une section transversale vue depuis une direction verticale, dans lequel :

l'imagerie tomographique de l'agrégat cellulaire est effectuée plusieurs fois à des intervalles de temps prédéterminés ;

l'efficacité médicamenteuse de la substance chimique est déterminée sur la base de changements dans le temps des quantités de caractéristiques calculées à partir des images tomographiques acquises par l'imagerie ; dans lequel

il est déterminé que l'efficacité médicamenteuse est démontrée sur la base d'une différence entre la courbure d'une surface de l'agrégat cellulaire dans une section transversale vue depuis la direction horizontale et la courbure d'une surface de l'agrégat cellulaire dans une section transversale vue depuis la direction verticale.

2. Procédé d'évaluation de l'efficacité selon la revendication 1, comprenant en outre

de détecter une cellule libre qui s'est détachée de l'agrégat cellulaire et s'est déposée sur la surface du fond du récipient sur la base des images tomographiques,

dans lequel l'efficacité de la substance chimique est déterminée sur la base d'un résultat de calcul de la quantité de caractéristiques et d'un résultat de détection de la cellule libre.

F I G.  1

F I G． 2 A

F I G． 2 B

F I G. 3

START

SET WELL PLATE TO
HOLDER SECTION — S101

SPHEROID IS IMAGED BY
TOMOGRAPHY — S102

GENERATE 3D IMAGE DATA — S103

CALCULATE APPROXIMATE
CURVED SURFACE — S104

CALCULATE FEATURE
AMOUNT — S105

END

FIG. 4A

FIG. 4B

FIG. 5

```
        ┌─────────────┐
        │    START    │
        └──────┬──────┘
               │
               ▼
    ┌────────────────────┐
    │  CULTURE CELL INSIDE│ ──── S201
    │ WELL, CREATE SPHEROID│
    └──────────┬─────────┘
               │
               ▼
    ┌────────────────────┐
    │ ADMINISTER CHEMICAL │
    │   SUBSTANCE TO BE   │ ──── S202
    │     EVALUATED       │
    └──────────┬─────────┘
               │
               ▼
    ┌────────────────────┐
    │ CARRY OUT IMAGING BY│
    │   IMAGE PROCESSING  │ ──── S203
    │     APPARATUS       │
    └──────────┬─────────┘
               │
               ▼
    ┌────────────────────┐
    │  EVALUATE MEDICAL   │
    │  EFFICACY BASED ON  │ ──── S204
    │  OUTPUT INFORMATION │
    └──────────┬─────────┘
               │
               ▼
        ┌─────────────┐
        │     END     │
        └─────────────┘
```

F I G. 6 A

F I G. 6 B

F I G. 6 C

F I G. 7 A

F I G. 7 B

F I G. 7 C

F I G. 7 D

FIG. 8A

FIG. 8B

FIG. 8C

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- JP 2011062166 A

- WO 2009107321 A

### Non-patent literature cited in the description

- **O.J. KLEIN et al.** Longitudinal, quantitative monitoring of therapeutic response in 3D in vitro tumor models with OCT for high-content therapeutic screening. *METHODS, US,* 15 March 2014, vol. 66 (2
- Cell Culture Imaging Using Microimpedance Tomography. **PONTUS LINDERHOLM et al.** IEEE TRANSACTIONS ON BIOMEDICAL ENGINEERING. IEEE, 01 January 2008, vol. 55

- **SRIRAM SUBRAMANIAM et al.** *Electron Thmography: a Powerful Tool for 3D Cellular Microscopy Technical refinements enable investigators to locate proteins inside cells and to anticipate tomographic reconstructions of bacteria,* 01 January 2008
- Imaging and Analysis of Three-Dimensional Cell Culture Models. **BENEDICT W. GRAF et al.** Methods in Molecular Biology. Humana Press, Inc, 24 October 2009, vol. 591, 211-227
- **SUSAN BRESLIN et al.** Three-dimensional cell culture: the missing link in drug discovery. *DRUG DISCOVERY TODAY,* 01 March 2013, vol. 18 (5-6